**Europäisches Patentamt**

(19) **European Patent Office**

**Office européen des brevets**

(11) Veröffentlichungsnummer: **0 024 516**
**B1**

(12) # EUROPÄISCHE PATENTSCHRIFT

(45) Veröffentlichungstag der Patentschrift:
28.07.82

(51) Int. Cl.³: **C 07 C 63/70**, C 07 C 51/58,
C 07 C 17/20, C 07 C 51/04

(21) Anmeldenummer: 80103922.3

(22) Anmeldetag: 09.07.80

(54) 4-Fluor-3-brombenzoylfluorid, Verfahren zu seiner Herstellung und zur Herstellung von 4-Fluor-3-brombenzoesäure daraus.

(30) Priorität: 18.07.79 DE 2928987

(43) Veröffentlichungstag der Anmeldung:
11.03.81 Patentblatt 81/10

(45) Bekanntmachung des Hinweises auf die Patenterteilung:
28.07.82 Patentblatt 82/30

(84) Benannte Vertragsstaaten:
BE CH DE FR GB IT LI NL

(56) Entgegenhaltungen:
EP-A-0 001 252
DE-A-2 062 434
DE-A-2 708 190
US-A-4 113 435

Chemical Abstracts Band 42, Nr. 15,
10 August 1948 Columbus, Ohio, USA
A. I. MASHENTSEV "Methods of synthesis of acyl fluorides directly from carboxylic acids», Spalte 5418 a–d & J. Applied Chem. (USSR) Band 20, 1947 Seiten 854 bis 863

Chemical Abstracts Band 40, Nr. 21,
10 November 1946 Columbus, Ohio, USA
A. I. MASHENTSEV "Fluorides of carboxylic acids. II. Methods of synthesis of benzoyl fluoride

(73) Patentinhaber: BAYER AG, Zentralbereich Patente, Marken und Lizenzen, D-5090 Leverkusen 1, Bayerwerk (DE)

(72) Erfinder: Marhold, Albrecht, Dr.,
Carl-Duisberg-Strasse 329, D-5090 Leverkusen (DE)
Erfinder: Kysela, Ernst, Dr., Auf dem Kamm 17,
D-5060 Bensberg (DE)
Erfinder: Klauke, Erich, Dr., Eichendorffweg 8,
D-5068 Odenthal (DE)

(56) Entgegenhaltungen:
and preparation of butyryl fluoride", Spalte 6443–6/7 & J. Gen. Chem. (USSR), Band 15, 1945 Seiten 915 bis 920

CHEMISCHES ZENTRALBLATT, Band 136, Nr. 17, 1965 Berlin
J.M.W. SCOTT «Die Orton-Umlagerung in geeigneten Lösungsmitteln. Einige Beobachtungen über die Reaktionen von p-substituierten N-Bromacylaniliden in Chlorbenzol und Trichloressigsäure»
Referat Nr. 895 & Canad. J. Chem. Band 38, 1960 Seiten 2441 bis 2449

## 4-Fluor-3-brombenzoylfluorid, Verfahren zu seiner Herstellung und zur Herstellung von 4-Fluor-3-brombenzoesäure daraus

Die Erfindung betrifft die Verbindung 4-Fluor-3-brombenzoylfluorid, sowie ein Verfahren zu seiner Herstellung.

Es wurde die Verbindung 4-Fluor-3-brom-benzoylfluorid gefunden.

Es wurde ferner ein Verfahren zur Herstellung von 4-Fluor-3-brom-benzoylfluorid gefunden, das dadurch gekennzeichnet ist, dass man p-Chlor-benzotrichlorid zunächst bromiert, dann an der Trichlormethylgruppe partiell hydrolysiert und das erhaltene Zwischenprodukt mit einem Fluorierungsmittel umsetzt.

Die Bromierung kann beispielsweise mit elementarem Brom, mit einer Brom abgebenden Verbindung, wie Sulfurylbromid oder Bromjod, oder mit einem Brom enthaltenden Komplex, wie Dioxan-Brom, ausgeführt werden.

Das p-Chlor-benzotrichlorid wird im erfindungsgemässen Verfahren im ersten Umsetzungsschritt bevorzugt mit elementarem Brom umgesetzt. Zur Vermeidung von Ausbeuteverlusten durch unvollständige Umsetzung ist es erforderlich, mindestens eine äquimolare Menge Brom, bevorzugt jedoch einen kleinen Überschuss, einzusetzen. Als Brommenge sei beispielsweise 1 bis 1,2, bevorzugt 1,03 bis 1,15 Mol Brom pro Mol p-Chlorbenzotrichlorid genannt.

Die Bromierung mit elementarem Brom wird in Gegenwart eines Stoffes durchgeführt, der die Kern-Bromierung einer aromatischen Verbindung fördert. Solche Stoffe sind beispielsweise die Halogenide, wie die Chloride, Bromide oder Jodide, von Aluminium, Eisen, Titan, Zink und Antimon, sowie Eisensulfid und Jod. Sollen als Halogenide der genannten Elemente die Bromide eingesetzt werden, so genügt es, die genannten Elemente in elementarer Form, beispielsweise als Pulver oder Späne, in das Reaktionsgemisch zu geben, wo sich dann durch Reaktion mit dem elementaren Brom die Bromide bilden. Beispiele für einzelne Stoffe, die die Kern-Bromierung aromatischer Verbindungen fördern, sind:

Aluminiumchlorid, Aluminiumbromid, Eisen-(III)-chlorid, Eisen-(III)-bromid, Titantetrachlorid, Titantetrabromid, Zinkchlorid, Zinkbromid, Antimon-(III)-chlorid, Antimon-(III)-bromid, Eisensulfid, Jod. Bevorzugt werden Eisen-Verbindungen, wie Eisen-(III)-chlorid, Eisen-(III)-bromid oder Eisensulfid, eingesetzt. Besonders bevorzugt wird Eisensulfid eingesetzt. Die genannten Stoffe zur Förderung der Kern-Bromierung aromatischer Verbindungen werden beispielsweise in einer Menge von 0,01 bis 3 Gewichtsprozent, bevorzugt 0,1 bis 1 Gewichtsprozent, bezogen auf die Menge Ausgangsprodukt, dem Reaktionsgemisch zugesetzt.

Die Bromierung im erfindungsgemässen Verfahren kann ohne Lösungsmittel durchgeführt werden. Es ist jedoch auch möglich, in Gegenwart eines inerten Lösungsmittels zu arbeiten. Als solches sei beispielsweise ein halogenierter Kohlenwasserstoff, wie Methylenchlorid, Chloroform, 1,2-Dichlorethan oder Trichlorethylen genannt.

Die erfindungsgemässe Bromierung wird bei schwach erhöhter Temperatur durchgeführt. Als solche sei beispielsweise eine Temperatur von 20 bis 70°C, bevorzugt von 30 bis 60°C, genannt.

Der zweite Umsetzungsschritt des erfindungsgemässen Verfahrens kann mit oder ohne Lösungsmittel durchgeführt werden. Für die Durchführung in Gegenwart eines Lösungsmittels seien inerte Lösungsmittel genannt. Beispiele hierfür sind halogenierte Kohlenwasserstoffe, wie Methylenchlorid, Chloroform, 1,2-Dichlorethan, Trichlorethylen oder Chlorbenzol, die bereits für den ersten Umsetzungsschritt des erfindungsgemässen Verfahrens genannt wurden.

Die erfindungsgemässe partielle Hydrolyse der Trichlormethylgruppe wird durch Zusatz von Wasser in einem zweiten Reaktionsschritt durchgeführt. Wasser kann beispielsweise als solches, oder als wässrige Lösung von Säuren oder Salzen zugesetzt werden. Bevorzugt ist der Zusatz von Wasser als solchem.

Der zweite Umsetzungsschritt des erfindungsgemässen Verfahrens wird weiterhin in Gegenwart eines Stoffes durchgeführt, der die partielle Hydrolyse der aromatisch gebundenen Trichlormethylgruppe fördert. Erfindungsgemäss können hierfür die gleichen Stoffe eingesetzt werden, die im ersten Umsetzungsschritt des erfindungsgemässen Verfahrens zur Förderung der Kern-Bromierung angewendet werden. Bevorzugt von den obengenannten Stoffen werden für den zweiten Umsetzungsschritt Eisen-Verbindungen eingesetzt. Ganz bevorzugt Eisenhalogenide, wie Eisen-(III)-chlorid oder Eisen-(III)-bromid. Der Stoff zur Förderung der partiellen Hydrolyse der aromatisch gebundenen Trichlormethylgruppe kann beispielsweise in einer Menge von 0,01 bis 3 Gewichtsprozent, bevorzugt 0,1 bis 1 Gewichtsprozent, bezogen auf das eingesetzte p-Chlorbenzotrichlorid, angewendet werden.

Das zur partiellen Hydrolyse im zweiten Umsetzungsschritt des erfindungsgemässen Verfahrens erforderliche Wasser wird so zum Reaktionsgemisch zudosiert, dass die exotherme Reaktion unter Kontrolle gehalten werden kann. Zur vollständigen Umsetzung im Sinne des erfindungsgemässen Verfahrens ist eine äquimolare Menge Wasser, bezogen auf das Ausgangsmaterial, erforderlich. Eine geringere als die äquimolare Menge Wasser führt zu einer unvollständigen Umsetzung und damit zu Ausbeuteverlusten. Zur Vervollständigung der Reaktion im zweiten Umsetzungsschritt des erfindungsgemässen Verfahrens wird daher etwas mehr als die äquimolare Menge, bezogen auf eingesetztes Ausgangsmaterial, verwendet. Als Wassermenge sei beispielsweise 1 bis 1,2 Mol Wasser, bevorzugt 1,05 bis 1,15 Mol Wasser, pro Mol Ausgangsverbindung genannt.

Das über die äquimolare Menge hinausgehende, im Reaktionsgemisch anwesende Wasser kann jedoch unerwünschte Nebenreaktionen verursachen. Erfindungsgemäss kann nunmehr das überschüssige, für die Reaktion des erfindungsgemässen Verfahrens nicht benötigte Wasser, durch Reaktion mit zugesetztem Thionylchlorid entfernt werden. Gleichzeitig ist es möglich, diejenigen Anteile des Reaktionsgemisches, die gegebenenfalls mit dem überschüssigen Wasser zu unerwünschten Nebenprodukten reagiert haben, mit Thionylchlorid zu dem im erfindungsgemässen Sinn bromierten und an der Trichlormethylgruppe partiell hydrolysierten p-Chlor-benzotrichlorid umzusetzen.

Als Temperatur für die partielle Hydrolyse sei beispielsweise 20 bis 100°C, bevorzugt 50 bis 85°C, genannt. Zur Beschleunigung der Hydrolyse-Reaktion kann es vorteilhaft sein, das Reaktionsgemisch anfangs etwas anzuheizen und erst danach die exotherme Reaktion durch die Geschwindigkeit der Wasserzugabe und/oder eine Kühlung des Reaktionsgemisches in dem gewünschten Temperaturbereich zu halten.

Im dritten Umsetzungsschritt des erfindungsgemässen Verfahrens wird das Umsetzungsprodukt des zweiten Schrittes mit einem Fluorierungsmittel wie Kaliumfluorid oder Caesiumfluorid weiter umgesetzt. Bevorzugt wird Kaliumfluorid als Fluorierungsmittel eingesetzt. Das Kaliumfluorid kann teilweise durch Natriumfluorid ersetzt werden und gegebenenfalls im Gemisch mit einem anderen Fluorierungsmittel angewandt werden.

Die erfindungsgemässe Fluorierung erfordert 2 Mol Kaliumfluorid pro Mol Ausgangsmaterial. Eine geringere Menge Kaliumfluorid verursacht Ausbeuteverluste durch unvollständige Reaktion. Ein geringer Überschuss an Kaliumfluorid ist daher für die Vervollständigung der Reaktion im erfindungsgemässen Verfahren vorteilhaft. Ein grösserer Überschuss an Kaliumfluorid ist unschädlich für den Erfolg des erfindungsgemässen Verfahrens, aus wirtschaftlichen Gründen jedoch nicht sinnvoll. Als Menge des Kaliumfluorids seien daher beispielsweise 2 bis 4 Mol Kaliumfluorid, bevorzugt 2,5 bis 3,5 Mol Kaliumfluorid, pro Mol eingesetztes p-Chlor-benzotrichlorid, genannt.

Es ist möglich, die Gesamtmenge Kaliumfluorid auf einmal zum Reaktionsgemisch für die dritte Umsetzungsstufe des erfindungsgemässen Verfahrens zuzusetzen. Es ist jedoch auch möglich, das Kaliumfluorid in mehreren, bevorzugt in zwei, Portionen zuzugeben. Bei dieser Variante wird ein Teil des Kaliumfluorids, bevorzugt etwa die Hälfte der Gesamtmenge, vor Beginn des dritten Umsetzungsschrittes zum Reaktionsgemisch gegeben, während der Rest in einer oder mehreren Portionen im Verlauf des dritten Umsetzungsschrittes des erfindungsgemässen Verfahrens zugefügt wird. Dieses gegebenenfalls im Verlaufe des dritten Umsetzungsschrittes zugegebene restliche Kaliumfluorid kann ohne weiteres Hilfsmittel oder in Form einer Suspension, bevorzugt in Form einer Suspension, eingesetzt werden. Als Suspensionsmittel kann gegebenenfalls ein Teil des für diesen dritten Umsetzungsschritt verwendeten Lösungsmittels benutzt werden.

Wird das Kaliumfluorid im dritten Umsetzungsschritt des erfindungsgemässen Verfahrens in mehreren Portionen zugesetzt, besteht weiterhin die Möglichkeit, den ersten Anteil oder die ersten Anteile Kaliumfluorid bis zu einer Menge von etwa 50 Mol-% des insgesamt zuzusetzenden Kaliumfluorids durch Natriumfluorid zu ersetzen. Da das Natriumfluorid gegenüber dem Kaliumfluorid preisgünstiger ist, ist diese Variante des dritten Umsetzungsschrittes bevorzugt.

Der dritte Umsetzungsschritt des erfindungsgemässen Verfahrens wird in einem inerten, polaren und aprotischen Lösungsmittel durchgeführt. Beispiele für solche Lösungsmittel sind: Tetramethylensulfon, Dimethylsulfon, N-Methylpyrrolidon. Bevorzugt wird als Lösungsmittel das Tetramethylensulfon verwendet.

Der dritte Umsetzungsschritt des erfindungsgemässen Verfahrens wird bei erhöhter Temperatur durchgeführt. Als solche sei beispielsweise eine Temperatur von 160 bis 220°C, bevorzugt 180 bis 220°C, genannt.

Die Reaktionsprodukte der einzelnen Umsetzungsschritte des erfindungsgemässen Verfahrens können nach Ablauf der einzelnen Reaktionen durch geeignete und dem Fachmann bekannte Methoden, wie Destillation oder Umkristallisation, aufgearbeitet werden und im nächsten Umsetzungsschritt eingesetzt werden. Erfindungsgemäss können jedoch die ersten beiden Umsetzungsschritte hintereinander ohne Isolierung des Zwischenproduktes durchgeführt werden. Bei dieser bevorzugten Variante des erfindungsgemässen Verfahrens wird im gleichen Reaktor zunächst die Reaktion des ersten Umsetzungsschrittes ohne Lösungsmittel durchgeführt. Danach wird unmittelbar anschliessend der zweite Umsetzungsschritt durchgeführt, zu dem in bevorzugter Weise eines der oben als geeignet beschriebene Lösungsmittel zugesetzt wird. Dieser bevorzugte Zusatz eines Lösungsmittels im zweiten Umsetzungsschritt ist vorteilhaft für die Beherrschung der exothermen Reaktion des zweiten Umsetzungsschrittes. Der zweite Umsetzungsschritt wird, wie weiter oben bereits beschrieben, bevorzugt in Gegenwart einer Eisen-Verbindung durchgeführt. Da eine der weiter oben beschriebenen Eisen-Verbindungen gleichermassen für den zweiten, wie auch für den ersten Umsetzungsschritt geeignet ist, erübrigt sich der Zusatz einer solchen Eisen-Verbindung im zweiten Umsetzungsschritt in dem Fall, wenn bereits im ersten Umsetzungsschritt in Gegenwart einer solchen Eisen-Verbindung gearbeitet wurde. In anderen Fällen kann eine solche geeignete Eisen-Verbindung nachträglich dem Reaktionsgemisch für den zweiten Umsetzungsschritt zugesetzt werden.

Das erfindungsgemässe Verfahren wird im allgemeinen wie folgt durchgeführt:

Das p-Chlor-benzotrichlorid, der zur Förderung der Kern-Bromierung aromatischer Verbindungen erforderliche Stoff und gegebenenfalls das Lösungsmittel werden in einer Rührapparatur vorgelegt und auf die gewünschte Temperatur erwärmt. Unter Rühren wird nun Brom im Verlaufe einiger Stunden zugesetzt. Nach einigen Stunden Nachrührzeit wird in der bevorzugten Variante eines der weiter oben als geeignet beschriebene Lösungsmittel zugesetzt und ebenfalls entsprechend der bevorzugten Variante eine der oben beschriebenen Eisen-Verbindungen zugegeben, falls diese nicht bereits aus dem ersten Umsetzungsschritt im Reaktionsgemisch enthalten ist. Sodann wird die für den zweiten Umsetzungsschritt gewünschte Temperatur eingestellt und Wasser so zugetropft, dass die gewählte Temperatur eingehalten werden kann.

Hierzu wird das Reaktionsgemisch gegebenenfalls in geeigneter Weise gekühlt. Das zugesetzte Wasser schliesst den weiter oben beschriebenen kleinen molaren Überschuss ein. Während des Zusatzes von Wasser entweicht Chlorwasserstoff aus dem Reaktionsgemisch und wird in einer geeigneten Absorptionsapparatur aufgefangen. Nach der Beendigung der Chlorwasserstoff-Entwicklung wird dem Reaktionsgemisch Thionylchlorid zugesetzt. Dessen Menge muss mindestens ausreichend sein, um das überschüssige Wasser zu entfernen und um die oben beschriebene Nebenprodukt-Bildung wieder rückgängig zu machen. Über diese Menge hinaus zugesetztes Thionylchlorid ist unschädlich für den Erfolg des erfindungsgemässen Verfahrens. Anschliessend wird das Lösungsmittel und das überschüssige Thionylchlorid abdestilliert und das gewünschte Reaktionsprodukt ebenfalls durch Destillation gewonnen. Zur dritten Umsetzungsstufe werden das Lösungsmittel, das Kaliumfluorid und das Reaktionsprodukt aus dem zweiten Umsetzungsschritt unter Rühren einige Stunden auf die gewählte Temperatur erwärmt. Das 4-Fluor-3-brom-benzoylfluorid wird durch destillative Trennung des Reaktionsgemisches des dritten Umsetzungsschrittes gewonnen. Bei der Variante des dritten Umsetzungsschrittes mit teilweisem Ersatz des Kaliumfluorids durch Natriumfluorid wird wie folgt verfahren: Das Reaktionsprodukt des zweiten Umsetzungsschrittes, Natriumfluorid in einer Menge, die 50 Mol-% der Gesamtmenge der Alkalifluoride entspricht, und etwa die Hälfte des Lösungsmittels werden unter Rühren einige Stunden auf die gewünschte Temperatur erwärmt. Anschliessend wird Kaliumfluorid in einer Menge, die den restlichen 50 Mol-% der Gesamtmenge an Alkalifluoriden entspricht, suspendiert in der Restmenge Lösungsmittel, zum Reaktionsgemisch gegeben. Nach einigen Stunden Nachrührzeit bei Reaktionstemperatur wird das Reaktionsgemisch wie oben beschrieben aufgearbeitet.

Das neue 4-Fluor-3-brom-benzoylfluorid ist im erfindungsgemässen Verfahren in hohen Ausbeuten über alle Umsetzungsschritte erhältlich. Es ist überraschend, dass alle Umsetzungsschritte des erfindungsgemässen Verfahrens ohne wesentliches Ausmass an Nebenreaktionen ablaufen, so dass kein vielfältiges Isomerengemisch, sondern ein durch einfache Destillation trennbares Reaktionsgemisch erhalten wird. Dieses überraschende Fehlen von erheblichen Nebenreaktionen gilt insbesondere für die dritte Umsetzungsstufe, bei der das Reaktionsgemisch bei der höheren Temperatur thermisch stark belastet wird.

Das neue 4-Fluor-3-brom-benzoylfluorid lässt sich alkalisch zur 4-Fluor-3-brom-benzoesäure verseifen. Diese Säure ist ein wichtiges Zwischenprodukt für die Herstellung hochwirksamer Insektizide. Dazu wird die 4-Fluor-3-brom-benzoesäure mit Natriumphenolat in die 4-Fluor-3-phenoxy-benzoesäure überführt, aus der dann durch Reduktion der entsprechende Benzylalkohol erhalten wird. Dieser lässt sich dann zum entsprechenden Benzaldehyd oxidieren, der mit Metallcyaniden zum α-Cyano-4-fluor-3-phenoxybenzylalkohol umgesetzt werden kann. Die Permethrinsäureester dieses Alkohols sind hervorragende Insektizide zur Bekämpfung von Schädlingen in der Landwirtschaft (DE-OS 27 09 264).

Die Schlüsselverbindung für die Herstellung der genannten Permethrinsäureester ist demnach die 4-Fluor-3-brom-benzoesäure. Diese ist bekannt und kann in einem vielstufigen und verlustreichen Herstellungsverfahren durch Oxidation von 4-Fluor-3-brom-toluol hergestellt werden, das wiederum durch Diazotierung von 2-Brom-4-methyl-anilin in Tetrafluoroborwasserstoff erhalten wird (Can. J. Chem. 31, 2444 [1960]). Dieses Verfahren zeigt einige Nachteile: So die Methode zur Einführung des Fluoratoms, bei der in Tetrafluoroborwasserstoff gearbeitet wird, der durch die Diazotierung wasserhaltig wird und damit korrosiv wirkt. Weiterhin ist die geringe Ausbeute bei der Oxidation des 4-Fluor-3-brom-toluols ein gravierender Nachteil. Und schliesslich ist als technisches Ausgangsmaterial das p-Aminotoluol anzusehen, das zuerst acyliert, dann bromiert und schliesslich vom Acetylrest wieder befreit werden muss, bevor die in Can. J. Chem. beschriebene Reaktionsfolge einsetzen kann.

Demgegenüber ist das Ausgangsprodukt des erfindungsgemässen Verfahrens, nämlich p-Chlor-benzotrichlorid, durch Chlorierung von p-Chlor-toluol leicht zugänglich (Houben-Weyl, Band V/3, Georg Thieme, Stuttgart 1962, S. 738) und das erfindungsgemässe Verfahren führt in wenigen ergiebigen Stufen zum erfindungsgemässen 4-Fluor-3-brom-benzoylfluorid, das praktisch quantitativ in die 4-Fluor-3-brom-benzoesäure übergeführt werden kann.

Ein weiteres Merkmal der vorliegenden Erfindung ist daher auch ein Verfahren zur Herstellung von 4-Fluor-3-brom-benzoesäure, das dadurch gekennzeichnet ist, dass man 4-Fluor-3-brom-benzoylfluorid zuerst mit einem alkalisch reagierenden Stoff umsetzt und dann aus dem entstandenen Salz mit Mineralsäure die 4-Fluor-3-brom-benzoesäure in Freiheit setzt.

Als alkalisch reagierender Stoff sei beispielsweise ein Alkalicarbonat oder Alkalihydroxid, wie Natriumcarbonat, Natriumhydrogencarbonat, Kaliumcarbonat, Kaliumhydrogencarbonat, Natriumhydroxid oder Kaliumhydroxid, bevorzugt Natriumhydroxid, genannt.

Als Mineralsäure sei beispielsweise konzentrierte oder verdünnte wässrige Salzsäure, Schwefelsäure, Phosphorsäure oder Salpetersäure, bevorzugt Salzsäure oder Schwefelsäure, genannt.

Die Herstellung von 4-Fluor-3-brom-benzoesäure aus 4-Fluor-3-brom-benzoylfluorid kann beispielsweise in Wasser als Reaktionsmedium bei einer Temperatur von 20 bis 100°C durchgeführt werden.

Beispiel 1

2776 g (12 Mol) p-Chlor-benzotrichlorid, 6 g gepulvertes Eisensulfid und 6 g wasserfreies Eisen-(III)-chlorid werden in einem 6-l-Dreihalskolben vorgelegt und unter Rühren bei 50°C mit 2200 g (13,75 Mol) Brom in 3 bis 4 Stunden versetzt. Man rührt 8 bis 10 Stunden nach, versetzt dann mit 3,2 l 1,2-Dichlorethan, erhöht die Temperatur auf 70°C und dosiert in ca. 3 bis 4 Stunden 248 g (13,75 Mol) Wasser zu. Sobald die Chlorwasserstoff-Entwicklung beendet ist, werden 200 ml Thionylchlorid zum Reaktionsgemisch gegeben. Nach Beendigung der Schwefeldioxid-Entwicklung werden das Lösungsmittel und das restliche Thionylchlorid abdestilliert und das 4-Chlor-3-brom-benzoylchlorid ebenfalls durch Destillation gewonnen. Man erhält 2730 g vom $Kp_{\cdot 0,5\ mbar}$: 98°C. Das entspricht 89% der theoretischen Ausbeute.

Für den dritten Umsetzungsschritt wird ein Teil des erhaltenen 4-Chlor-3-brom-benzoylchlorids, nämlich 1825 g (7 Mol) mit 3250 g Tetramethylensulfon und 1250 g (21 Mol) wasserfreiem Kaliumfluorid unter Rühren 7 Stunden auf 210°C erwärmt. Die anschliessende Destillation liefert 1330 g 4-Fluor-3-brom-benzoylfluorid vom $Kp_{18\ mbar}$: 92–95°C. Das entspricht 83% der theoretischen Ausbeute.

Beispiel 2

Die ersten beiden Umsetzungsschritte werden wie im Beispiel 1 durchgeführt. Anschliessend werden 1270 g (5 Mol) 4-Chlor-3-brom-benzoylchlorid mit 1150 g Tetramethylensulfon und 315 g (7,5 Mol) wasserfreiem Natriumfluorid unter Rühren 5 Stunden auf 210°C erwärmt. Danach wird das Reaktionsgemisch mit 435 g (7,5 Mol) wasserfreiem Kaliumfluorid, das in 1150 g Tetramethylensulfon suspendiert ist, versetzt und weitere 7 Stunden bei 210°C gerührt. Die anschliessende Destillation liefert 873 g 4-Fluor-3-brom-benzoylfluorid. Das entspricht 79% der theoretischen Ausbeute.

Beispiel 3

Man legt 100 g Natriumhydroxyd in 1200 ml Wasser vor und tropft unter Rühren 265 g 4-Fluor-3-brom-benzoylfluorid zu. Wenn nach Ende der Zugabe alles gelöst ist, wird mit konz. Salzsäure sauer gestellt. Das ausgefallene Produkt wird abgesaugt und bei 100°C in einem Umluftschrank getrocknet. Man erhält eine Ausbeute von 252 g 4-Fluor-3-brom-benzoesäure, das entspricht 96% der theoretischen Ausbeute; Fp.: 134-136°C.

**Patentansprüche**

1. Die Verbindung 4-Fluor-3-brom-benzoylfluorid.

2. Verfahren zur Herstellung von 4-Fluor-3-brom-benzoylfluorid, dadurch gekennzeichnet, dass man p-Chlor-benzotrichlorid zunächst bromiert, dann an der Trichlormethylgruppe partiell hydrolysiert und das erhaltene Zwischenprodukt mit einem Fluorierungsmittel umsetzt.

3. Verfahren nach Anspruch 2, dadurch gekennzeichnet, dass man die Bromierung mit elementarem Brom in Gegenwart eines Stoffes, der die Kern-Bromierung aromatischer Verbindungen fördert, sowie gegebenenfalls in Gegenwart eines inerten Lösungsmittels durchführt.

4. Verfahren nach Anspruch 2 und 3, dadurch gekennzeichnet, dass als Stoff, der die Kern-Bromierung aromatischer Verbindungen fördert, Eisen in elementarer oder gebundener Form eingesetzt wird.

5. Verfahren nach Anspruch 2, dadurch gekennzeichnet, dass man die partielle Hydrolyse mit Wasser und in Gegenwart eines Stoffes, der die Hydrolyse der aromatisch gebundenen Trifluormethylgruppe fördert, sowie gegebenenfalls in Gegenwart eines inerten Lösungsmittels durchführt, wobei man gegebenenfalls überschüssiges Wasser und/oder durch überschüssiges Wasser gebildete Nebenprodukte durch Umsetzung mit Thionylchlorid entfernt.

6. Verfahren nach Anspruch 2 bis 5, dadurch gekennzeichnet, dass man die Bromierung und die partielle Hydrolyse im selben Reaktor ohne zwischengeschaltete Aufarbeitung durchführt.

7. Verfahren nach Anspruch 2, dadurch gekennzeichnet, dass man zur Umsetzung mit einem Fluorierungsmittel Kaliumfluorid, welches teilweise durch Natriumfluorid ersetzt sein kann, in Gegenwart eines inerten Lösungsmittels bei erhöhter Temperatur einsetzt.

8. Verfahren nach Anspruch 2 und 7, dadurch gekennzeichnet, dass man das Kaliumfluorid in mehreren Portionen zusetzt.

9. Verfahren nach Anspruch 2, 7 und 8, dadurch gekennzeichnet, dass man zunächst Natriumfluorid in einer ersten Portion und dann Kaliumfluorid in einer zweiten Portion zusetzt.

10. Verfahren zur Herstellung von 4-Fluor-3-brombenzoesäure, dadurch gekennzeichnet, dass man 4-Fluor-3-brom-benzoylfluorid zuerst mit einem alkalisch reagierenden Stoff umsetzt und dann aus dem entstandenen Salz mit Mineralsäure die 4-Fluor-3-brom-benzoesäure in Freiheit setzt.

Claims

1. The compound 4-fluoro-3-bromo-benzoyl fluoride.

2. Process for the preparation of 4-fluoro-3-bromo-benzoyl fluoride, characterised in that p-chloro-benzotrichloride is first brominated, the trichloromethyl group is then partially hydrolysed and the resulting intermediate product is reacted with a fluorinating agent.

3. Process according to Claim 2, characterised in that the bromination is carried out with elementary bromine in the presence of a substance which promotes bromination of the nucleus of aromatic compounds, and if appropriate in the presence of an inert solvent.

4. Process according to Claim 2 and 3, characterised in that iron in elementary or bonded form is employed as the substance which promotes bromination of the nucleus of aromatic compounds.

5. Process according to Claim 2, characterised in that the partial hydrolysis is carried out with water and in the presence of a substance which promotes hydrolysis of the aromatically bonded trifluoromethyl group, and if appropriate in the presence of an inert solvent, excess water and/or by-products formed by excess water being removed, if appropriate, by reaction with thionyl chloride.

6. Process according to Claim 2 to 5, characterised in that the bromination and the partial hydrolysis are carried out in the same reactor, without intermediate working up.

7. Process according to Claim 2, characterised in that, for the reaction with a fluorinating agent, potassium fluoride, some of which can be replaced by sodium fluoride, is employed in the presence of an inert solvent and at elevated temperature.

8. Process according to Claim 2 and 7, characterised in that the potassium fluoride is added in several portions.

9. Process according to Claim 2, 7 and 8, characterised in that sodium fluoride is initially added in a first portion and potassium fluoride is then added in a second portion.

10. Process for the preparation of 4-fluoro-3-bromobenzoic acid, characterised in that 4-fluoro-3-bromobenzoyl fluoride is first reacted with an alkaline substance and 4-fluoro-3-bromo-benzoic acid is then liberated from the resulting salt by means of mineral acid.

Revendications

1. Le fluorure de 4-fluoro-3-bromobenzoyle.

2. Procédé pour la préparation de fluorure de 4-fluoro-3-bromobenzoyle, caractérisé en ce que l'on brome d'abord le chlorure de p-chlorobenzényle, on l'hydrolyse ensuite partiellement sur le groupe trichlorométhyle et l'on fait réagir le produit intermédiaire obtenue avec un agent de fluoruration.

3. Procédé selon la revendication 2, caractérisé en ce que l'on effectue la bromuration par le brome élémentaire en présence d'un composé qui accélère la bromuration au noyau d'un composé aromatique, ainsi éventuellement qu'en présence d'un solvant inerte.

4. Procédé selon les revendications 2 et 3, caractérisé en ce qu'on utilise le fer sous forme élémentaire ou sous forme liée comme substance accélérant la bromuration au noyau des composés aromatiques.

5. Procédé selon la revendication 2, caractérisé en ce que l'on effectue l'hydrolyse partielle par l'eau et en présence d'une substance qui accélère l'hydrolyse du groupe trifluorométhyle à liaison aromatique, ainsi éventuellement qu'en présence d'un solvant inerte, en éliminant éventuellement par réaction avec le chlorure de thionyle l'eau en excès et/ou les sous-produits formés par l'eau en excès.

6. Procédé selon les revendications 2 à 5, caractérisé en ce que l'on effectue la bromuration et l'hydrolyse partielle dans le même réacteur sans traitement intermédiaire.

7. Procédé selon la revendication 2, caractérisé en ce que l'on utilise pour la réaction avec un agent de fluoruration, le fluorure de potassium qui peut être partiellement remplacé par le fluorure de sodium, en présence d'un solvant inerte à température élevée.

8. Procédé selon les revendications 2 et 7, caractérisé en ce que l'on ajoute le fluorure de potassium en plusieurs portions.

9. Procédé selon les revendications 2, 7 et 8, caractérisé en ce que l'on ajoute d'abord le fluorure de sodium dans une première portion et ensuite le fluorure de potassium dans une seconde portion.

10. Procédé pour la préparation d'acide 4-fluoro-3-bromobenzoïque, caractérisé en ce que l'on fait réagir d'abord le fluorure de 4-fluoro-3-bromobenzoyle avec une substance à réaction alcaline et ensuite on libère l'acide 4-fluoro-3-bromobenzoïque du sel formé au moyen d'un acide minéral.